# EUROPÄISCHE PATENTSCHRIFT

(11) **EP 2 942 070 B1**
(45) Veröffentlichungstag und Bekanntmachung des Hinweises auf die Patenterteilung: **10.01.2018**
(21) Anmeldenummer: 15164010.9
(22) Anmeldetag: 17.04.2015
(51) Int. Cl.: A61M 1/16, F16L 59/14, F16L 59/153

(54) **SCHLAUCHLEITUNG FÜR FRISCHE UND/ODER VERBRAUCHTE DIALYSIERFLÜSSIGKEIT**
HOSE LINE FOR FRESH AND/OR CONSUMED DIALYSIS FLUID
CONDUITE EN TUYAUX FLEXIBLES POUR LIQUIDES DE DIALYSE USAGÉS OU FRAIS

(30) Priorität: 08.05.2014 DE 102014106490
(43) Veröffentlichungstag der Anmeldung: 11.11.2015
(73) Patentinhaber: B. Braun Avitum AG, 34212 Melsungen (DE)
(72) Erfinder: Ritter, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- EP-A1- 0 499 718
- WO-A1-01/13021
- WO-A1-2014/118168
- DE-U1- 29 917 247
- DE-U1-202005 021 496
- US-A- 3 712 475
- US-A- 5 861 555
- US-A1- 2003 143 352
- US-A1- 2013 056 419
- US-B2- 6 821 421

## Beschreibung

Die Erfindung betrifft eine Schlauchverbindung zur Verwendung im Rahmen einer Blutreinigung, und bezieht sich vorzugsweise auf einen Schlauch zur Durchleitung von Dialysierflüssigkeit bei Verwendung mit einer Dialysemaschine.

Derzeit werden bei nahezu allen auf dem Markt befindlichen Dialysemaschinen an deren Aus- und/oder Eingängen bzw. an einem entsprechenden Zulauf bzw. Ablauf für Dialysierflüssigkeit Schlauchleitungen oder Schläuche mit sogenannten Hansen-Kupplungen zum Anschluss an einen Dialysator eingesetzt. Während eines Therapieprozesses ist der Dialysator an die Dialysemaschine konnektiert, und strömt die Dialysierflüssigkeit durch das Gehäuse des Dialysators, während zu reinigendes Blut im Gegenfluss durch Hohlfasern im Inneren des Dialysators strömt.

Beispielsweise während eines Desinfektionsprozesses werden die Schlauchleitungen unter Abkopplung des Dialysators kurzgeschlossen, wozu sie meist so an die Dialysemaschine angekoppelt werden, dass die beiden Leitungen intern verbunden sind. Darüber hinaus sind auch sogenannte Spülbrücken bekannt, bei deren Verwendung die entsprechenden Hansen-Kupplungen mittels eines externen Adapters miteinander verbunden werden.

In der Regel werden flexible Schlauchleitungen, beispielsweise solche aus Silikon (mit oder ohne Gewebeverstärkung), bevorzugt. Andere Materialien, die hinreichend Flexibilität bereitstellen, sind möglich. Der Innendurchmesser der Schlauchleitungen beträgt typisch 5mm, bei einer Wandstärke der Schlauchleitung von 2 bis 3 mm. Die Strömungsmenge der Dialysierflüssigkeit liegt bei diesen Schlauchleitungen zwischen 100 ml/min und 1000 ml/min. Insbesondere ist der Innendurchmesser der Schlauchleitungen so ausgelegt, dass bei der höchsten Flussrate nur ein niedriger Gegendruck entsteht. Die genannten Schläuche sind in der Regel semitransparent bis opak, so dass eine Rotfärbung der Dialysierflüssigkeit, etwa bei Auftreten eines Blutverlusts, oder Luftblasen erkannt werden können.

Neuere Normen auf dem Gebiet der Blutreinigung, beispielsweise die chinesische Norm YY0054-2010 für Hämodialysegeräte, fordern eine Temperaturgenauigkeit von +/-0,5 °C am dialysatorseitigen Ende des zum Dialysator führenden Schlauchs (an der Kupplung zum Dialysator gemessen).

Die Temperaturregelung der Dialysierflüssigkeit erfolgt über Sensoren im Inneren der Dialysemaschine in der Nähe des Anschlusses des Zuleitungsschlauchs. Stromab strömt die Dialysierflüssigkeit sodann durch einen bezüglich der Dialysemaschine außen liegenden Schlauchabschnitt zum Eingang/Einlass des Dialysators. Die Länge dieses außen liegenden Schlauchs oder Schlauchabschnitts beträgt typisch 0,80 m bis 1 m. Die Umgebungstemperatur beträgt typisch zwischen 10 °C bis 40 °C und in der Regel zwischen 15 °C und 35 °C (Zimmertemperatur). Infolgedessen wird die Temperatur der Dialysierflüssigkeit durch die Umgebungstemperatur entlang des Schlauchabschnitts zwischen dem Ausgang an der Dialysemaschine und der eingangsseitigen Kupplung am Dialysator beeinflusst bzw. verändert (meist verringert, bei bestimmten Konstellationen, wie z.B. niedriger Dialysierflüssigkeitstemperatur und hoher Umgebungstemperatur auch erhöht).

Die Druckschrift EP 0499718 A1 beispielsweise offenbart eine Anordnung von Schläuchen zur Peritonealdialyse, mit einem zu einem Patienten führenden Schlauchabschnitt zur Zufuhr frischer Dialyselösung in den Peritonealraum des Patienten und zur Ausleitung verbrauchter Dialyselösung aus dem Peritonealraum des Patienten. Der bekannte Schlauchabschnitt weist dazu mehrere Lumen derart auf, dass durch wenigstens ein erstes der mehreren Lumen frische Lösung zugeführt und durch wenigstens ein zweites der mehreren Lumen verbrauchte Lösung abgeführt wird. Die einzelnen Lumen münden distal bzw. vom Körper des Patienten weg gerichtet in einem T-förmigen Verbinder, der die Fluidströme in den Lumen auf jeweils weiterführende Leitungsabschnitte für den Zulauf frischer Lösung und den Ablauf verbrauchter Lösung aufteilt.

Die Druckschrift DE 20 2005 021 196 U1 zeigt eine Vorrichtung zum Temperieren einer Fluidleitung mit einer Mantelleitung, die einen Längsschlitz aufweist, an dem die Fluidleitung in einen Hohlraum der Mantelleitung einsetzbar ist, wobei sich in Längsrichtung der Mantelleitung mindestens ein Heizelement erstreckt.

Die Druckschrift DE 229 17 247 U1 beschreibt eine Wärmeisolations- oder Wärmeübertragungskörper für Infusionsschläuche zur Anbringung auf der Außenumfangsfläche eines von einer Infusionslösung durchströmbaren Infusionsschlauches.

Die Druckschrift US 2003/0143352 A1 zeigt eine mehrlumige Schlauchquerschnittsgemeometrie eines Schlauchs für eine Peritonealdialyse.

Die Druckschrift WO 01/13021 A1 zeigt eine mehrlumige Schlauchquerschnittsgeometrie eines Schlauchs für einen Infusionsschlauch.

Die Druckschrift US 2013/0056419 A1 beschreibt eine Dialysemaschine mit Leitungen unterschiedlichen Querschnitts.

Die Druckschrift US 3 712 475 A zeigt eine Abdeckung für ein Dialysegerät mit einem Drehventil zum Regulieren eines Leitungsquerschnitts.

Die Druckschrift US 6 821 421 B1 offenbart Hämodialyseeinheit mit Schlauchabschnitten unterschiedlichen Querschnitts.

Die Druckschrift WO 2014/118168 A1 beschreibt eine Vorrichtung zur Regulierung eines Ultrafiltrationsverfahrens mit einem Drosselventil zum Regulieren der Durchflussrate.

Ferner ist aus der Druckschrift JP 2001-340466 A, veröffentlicht auch als Druckschrift JP 4-567847 B2, ein Doppellumenkatheter mit einem Fluidzufuhrlumen und einem Fluidrückführvolumen, die durch eine Trennwand voneinander getrennt sind, innerhalb des Katheters bekannt.

Nachteilig bei den bekannten Mehrlumenstrukturen ist, dass die Leitungs- oder Kanalabschnitte nicht isoliert sind. Insbesondere bei niedrigen Strömungsraten der Dialysierflüssigkeit, d.h. bei langsamem Fluss bzw. langsamer Strömung der Dialysierflüssigkeit, kann daher die wie vorstehend erwähnt geforderte Temperaturkonstanz der Dialysierflüssigkeit aufgrund des Einflusses der Umgebungstemperatur nicht sicher erreicht werden.

So kann bei geringer und damit die Dialysierflüssigkeit kühlender Umgebungstemperatur die Temperatur der Dialysierflüssigkeit am Dialysatoreingang unerwünscht zu gering sein. Eine Kompensation auftretender Temperaturverluste ist jedoch nicht möglich, da die Verluste entlang der Schlauchleitung nicht genauer bekannt sind. Außerdem kann im Desinfektionsbetrieb der Temperaturverlust heißen Wassers zu hoch sein, so dass die zur Desinfektion notwendige Temperatur, beispielsweise 83°C, nicht auf der gesamten Desinfektionsstrecke sichergestellt werden kann. Es entsteht ein zusätzlicher Energieverbrauch durch ein erforderliches Nachheizen zum Ausgleich von Wärmeverlusten in die Umgebung. Schließlich besteht ohne Isolation bei einer Berührung während der Desinfektion Verbrennungsgefahr.

Andererseits kann bei hoher und damit die Dialysierflüssigkeit erwärmender Umgebungstemperatur die Temperatur der Dialysierflüssigkeit am Dialysatoreingang unerwünscht zu hoch sein.

Weiter nachteilig kommt es durch Temperaturschwankungen der Dialysierflüssigkeit zu Masseänderungen in derselben, welche aufgrund der volumetrischen Bilanzierung der Dialysemaschine zu Bilanzierungsfehlern bzw. Bilanzierungsabweichungen im Gewicht führen. In einer Beispielrechnung mit 37 °C und einer Dichte von 993, 32 der Dialysierflüssigkeit im Hinlauf und einer darauf bezogen um 1°C, 2°C oder 3°C geringeren Rücklauftemperatur (36°C, 35°C oder 34°C), entsprechend einer Dichte von 993,68, 994,03 bzw. 994,37 im Dialysierflüssigkeitsrücklauf, ermitteln sich bei einem Fluss von 800 ml/min bezogen auf 4 Stunden bilanzierte Gewichtsabweichungen von 69,1 g, 136,3 g bzw. 201,6 g.

### Kurze Beschreibung der Erfindung

Der Erfindung liegt daher als eine Aufgabe zugrunde, die vorgenannten Probleme bekannter Anordnungen zu lösen und einen Dialysierflüssigkeitsschlauch zu schaffen, der unter Umgebungstemperatureinflüssen eine verbesserte Temperaturkonstanz darin strömender frischer Dialysierflüssigkeit bis zu einem dialysatorseitigen Eingang und/oder darin strömender verbrauchter Dialysierflüssigkeit bis zu einem dialysemaschineseitigen Eingang bereitstellt.

Diese Aufgabe wird erfindungsgemäß durch die Merkmalskombination des Anspruchs 1 gelöst. Vorteilhafte Weiterbildungen der Erfindung sind Gegenstand der beigefügten Unteransprüche.

Ein Grundgedanke der Erfindung besteht somit darin, durch geeignete (Schlauchgeometrie - bewirkte) Isolation und/oder geeignet mehrlumigen Aufbau einer Schlauchleitung für Dialysierflüssigkeit Temperaturverluste von darin strömender Dialysierflüssigkeit in die Umgebung zu verringern. In anderen Worten ausgedrückt beruht die vorliegende Erfindung auf dem Grundkonzept, eine Schlauchquerschnittsgeometrie zu schaffen, die zusätzlich zu einem vorzugsweise zentralen Hauptströmungskanal oder Hauptlumen wenigstens einen weiteren Nebenströmungskanal oder Nebenlumen zur Isolation des Hauptlumens und/oder für die wahlweise Zusatz-Fluidförderung in die gleiche Richtung wie der Hauptströmungskanal vorsieht. Auf diese Weise ist es möglich in Abhängigkeit des beabsichtigten Strömungsvolumens (der beabsichtigten Strömungsrate) den optimalen aktiven Kanalgesamtquerschnitt gemäß dem Haupt- und/oder Nebenströmungskanal zu wählen und frei zuschalten. Dabei kann der wenigstens eine Nebenströmungskanal im Querschnitt zumindest teilkreisförmig um den Hauptströmungskanal angeordnet sein, um zumindest in inaktivem (nicht frei geschaltetem) Zustand eine Art Luftposter zwischen dem Hauptströmungskanal und der Umgebung zu schaffen, welches als Wärmeisolation dient.

Es wird im Konkreteren vorgeschlagen, in ggf. Kombination mit einer bereits vorhandenen Isolation der Schlauchleitung mehrere Lumen oder Kanäle jeweils gleichen oder alternativ unterschiedlichen Querschnitts innenseitig der Schlauchleitung so vorzusehen, dass für einen jeweils auftretenden Fluss und/oder eine jeweilige Umgebungstemperatur ein jeweils optimaler Querschnitt bzw. Gesamtquerschnitt verwendet werden kann. D.h. es wird ein Dialysierflüssigkeitsschlauch oder DF-Schlauch vorgeschlagen, mit einem mehrlumigen Querschnitt, der so aufgebaut ist, dass der Temperaturverlust zwischen Spülbrücke und Dialysator minimierbar ist. Dazu sind insbesondere zwei miteinander kombinierbare Mittel, zum einen die Isolationsfunktion des Schlauchs vorzugsweise durch Ausbildung von Nebenlumen (Nebenströmungskanälen) radial um das Hauptlumen (Hauptströmungskanal) und/oder zum anderen die Bereitstellung mehrerer individuell freischaltbarer Lumen (Haupt-/ und Nebenlumen) in einem Schlauch, so dass jeweils der optimale Schlauchquerschnitt für den jeweiligen Fluss (Strömungsvolumen/Strömungsrate) gewählt werden kann, vorgesehen.

Insgesamt werden erfindungsgemäß Vorteile dahingehend erzielt, dass der Temperaturverlust der Dialysierflüssigkeit außerhalb der Dialysemaschine in Richtung der Erfüllung bestehender Norman reduziert wird, dass die Genauigkeit der Temperatur der Dialysierflüssigkeit am Dialysator verbessert wird, und eine Verringerung der temperaturbedingten Masseänderung und dadurch eine Verbesserung der Ultrafiltrationsgenauigkeit erzielt wird. Ein weiterer Vorteil besteht in einer einfachen und kostengünstigen Nachrüstbarkeit bestehender Maschinen und Vorrichtungen.

Die Aufgabe wird somit gelöst durch eine Dialysierflüssigkeits-Schlauchleitung zur Herstellung einer Fluidverbindung zwischen einem Dialysierflüssigkeits-Ausgang an einer Dialysemaschine und einem Dialysierflüssigkeits-Eingang an einem Dialysator und/oder Herstellung einer Fluidverbindung zwischen einem Dialysierflüssigkeits-Ausgang an dem Dialysator und einem Dialysierflüssigkeits-Eingang an der Dialysemaschine, gekennzeichnet durch wenigstens ein zur Durchleitung von ausschließlich Dialysierflüssigkeit ausgelegtes (Haupt-)Lumen eines vorbestimmten Querschnitts und/oder Durchmessers und wenigstens ein weiteres (Neben-)Lumen eines vorbestimmten Querschnitts und/oder Durchmessers vorzugsweise unterschiedlich zum wenigstens einen (Haupt-)Lumen, wobei das wenigstens eine (Haupt-)Lumen und weitere (Neben-)Lumen dazu angeordnet sind, je nach deren wahlweise individuelle Freischaltung einen gesamten Dialysierflüssigkeitsfluss derart bereitzustellen, dass ein Temperaturverlust der Dialysierflüssigkeit entlang der Fluidstrecke zwischen Dialysemaschine und Dialysator begrenzt und eine vorbestimmte Temperaturgenauigkeit der Dialysierflüssigkeit zumindest am Dialysierflüssigkeits-Eingang des Dialysators erhalten wird.

Anders ausgedrückt, wird die Aufgabe gelöst durch eine Dialysierflüssigkeits-Schlauchleitung zur Herstellung einer Fluidverbindung zwischen einem Dialysierflüssigkeits-Ausgang an einer Dialysemaschine und einem Dialysierflüssigkeits-Eingang an einem Dialysator und/oder zur Herstellung einer Fluidverbindung zwischen einem Dialysierflüssigkeits-Ausgang an dem Dialysator und einem Dialysierflüssigkeits-Eingang an der Dialysemaschine, mit
einer mehrlumigen Schlauchquerschnittsgeometrie, die zusätzlich zu einem Hauptlumen, wenigstens ein weiteres Nebenlumen aufweist,
dadurch gekennzeichnet, dass
das Hauptlumen einen Hauptströmungskanal ausbildet, und
das wenigstens eine weitere Nebenlumen als Isolationskammer zur umfangsseitigen Wärmeisolation des Hauptlumens und/oder als Nebenströmungskanal für die wahlweise alternative oder zusätzliche Dialysierflüssigkeitsförderung in die gleiche Richtung wie das Hauptlumen angeordnet ist.

Vorteilhaft lässt sich mit einer Dialysierflüssigkeits-Schlauchleitung diesen Aufbaus der Temperaturverlust der Dialysierflüssigkeit, aber auch die Temperaturerhöhung der Dialysierflüssigkeit außerhalb der Dialysemaschine reduzieren. Ferner wird eine Verbesserung der Genauigkeit der Temperatur der Dialyseflüssigkeit am Dialysator erreicht. Außerdem wird eine Reduktion der temperaturbedingten Masseänderung und dadurch eine Verbesserung der Ultrafiltrationsgenauigkeit erzielt. Zudem ist diese Dialysierflüssigkeits-Schlauchleitung bei bestehenden Systemen effizient nachrüstbar.

Bevorzugt wird bei der Dialysierflüssigkeits-Schlauchleitung, dass das wenigstens eine Lumen eine Wandung aus einem flexiblen und/oder transparenten oder opaken Material aufweist, und die Wandung außenseitig von einer Isolationsstruktur umgeben ist, in welcher Luft einen Wärmeübergang aus der Dialysierflüssigkeit in die Umgebung der Schlauchleitung verringert.

Eine flexible Schlauchleitung erleichtert die Handhabung der Schlauchleitung, und ein transparentes oder opakes, d.h. milchig durchscheinendes Material der Schlauchwandung erleichtert vorteilhaft die Erkennbarkeit von Blutverlusten während eines Therapieprozesses und/oder die Erkennung unerwünschter Luftblasen in der Dialysierflüssigkeit.

Weiter bevorzugt wird bei der Dialysierflüssigkeits-Schlauchleitung, dass die Isolationsstruktur aus einem extrudierten und/oder geschäumten, flexiblen Material oder aus demselben Material wie die Wandung des wenigstens einen Lumens besteht. Dadurch lässt sich die Isolationsstruktur auf einfache Weise auf eine bestehende Schlauchleitung aufbringen oder bereits während der Herstellung der Schlauchleitung mit erzeugen.

Außerdem bevorzugt wird bei der Dialysierflüssigkeits-Schlauchleitung, dass die Isolationsstruktur kammerförmige oder kreisförmige, luftgefüllte Kanalstrukturen aufweist, die durch stegartiges, radial nach außen verlaufendes Material der Wandung und/oder der Isolationsstruktur voneinander getrennt und äquidistant in der Isolationsstruktur verteilt sind. Hierdurch werden vorteilhaft umlaufend und in Längsrichtung der Schlauchleitung gleichförmige Isolationseigenschaften erzielt.

In einer bevorzugten Ausführungsform ist bei der Dialysierflüssigkeits-Schlauchleitung der Schlauchquerschnitt in wenigstens zwei Lumen (Haupt- und Nebenlumen) gleichen oder unterschiedlichen Querschnitts und/oder Durchmessers aufgeteilt, wobei die wenigstens zwei Lumen bei Beaufschlagung mit Dialysierflüssigkeit von einer eingangsseitig externen Vorrichtung an der Dialysemaschine einzeln oder in paralleler Weise mit einem Dialysierflüssigkeitsfluss beaufschlagbar sind, und auslassseitig bzw. ausgangseitig am Dialysator in ein Einzellumen münden, und die wenigstens zwei Lumen gleichen oder unterschiedlichen Querschnitts und/oder Durchmessers dazu angeordnet sind, einen Dialysierflüssigkeitsfluss in die gleiche Richtung durch wenigstens eines der wenigstens zwei Lumen derart variabel bereitzustellen, dass ein Temperaturverlust der Dialysierflüssigkeit entlang der Fluidstrecke zwischen Dialysemaschine und Dialysator begrenzt und eine vorbestimmte Temperaturgenauigkeit der Dialysierflüssigkeit am Dialysierflüssigkeits-Eingang des Dialysators erhalten wird.

Vorteilhaft lässt sich mit einer Dialysierflüssigkeits-Schlauchleitung der Temperaturverlust der Dialysierflüssigkeit außerhalb der Dialysemaschine nochmals verbessert reduzieren. Ferner wird eine weitere Verbesserung der Genauigkeit der Temperatur der Dialyseflüssigkeit am Dialysator erreicht. Außerdem wird ebenfalls eine Reduktion der temperaturbedingten Masseänderung und dadurch eine Verbesserung der Ultrafiltrationsgenauigkeit erzielt. Zudem ist auch diese Dialysierflüssigkeits-Schlauchleitung bei bestehenden Systemen effizient nachrüstbar.

Auch bevorzugt weisen die wenigstens zwei Lumen jeweils denselben Querschnitt und/oder Durchmesser auf und sind einzeln für einen Dialysierflüssigkeitsfluss einer ersten Rate oder parallel gleichzeitig für einen Dialysierflüssigkeitsfluss einer zweiten Rate (in die gleiche Strömungsrichtung) schaltbar.

Alternativ bevorzugt weisen die wenigstens zwei Lumen jeweils unterschiedlichen Querschnitt und/oder Durchmesser auf und sind einzeln für einen Dialysierflüssigkeitsfluss einer dem jeweiligen Einzellumen entsprechenden Rate oder parallel gleichzeitig für einen Dialysierflüssigkeitsfluss einer Gesamtrate schaltbar.

Weiter alternativ bevorzugt weisen die wenigstens zwei Lumen jeweils unterschiedlichen Querschnitt und/oder Durchmesser auf und sind einzeln für einen Dialysierflüssigkeitsfluss einer dem jeweiligen Einzellumen entsprechenden Rate oder parallel gleichzeitig für einen Dialysierflüssigkeitsfluss einer Gesamtrate schaltbar, wobei ein Lumen kleineren Querschnitts und/oder Durchmessers innerhalb eines Lumens größeren Querschnitts und/oder Durchmessers angeordnet ist und durch Material der Wandung von dem Lumen größeren Querschnitts und/oder Durchmessers getrennt ist.

Vorteilhaft bei Verwendung mehrerer Lumen an bzw. in einer Schlauchleitung ist, dass jeweils ein optimaler Querschnitt für einen jeweiligen Fluss von Dialysierflüssigkeit verwendet werden kann. Unter dem Begriff schaltbar ist vorzugsweise auch wählbar zu verstehen. Durch Schalten bzw. Aufschalten eines Flusses oder Stroms von frischer oder verbrauchter Dialysierflüssigkeit auf nur ein Lumen (wobei dann die verbleibenden Lumen nicht durchströmt werden) oder auf parallel mehrere Lumen bis hin zu parallel allen Lumen der Schlauchleitung mittels einer an einem Ende der Schlauchleitung angeordneten Vorrichtung, beispielsweise einem manuell oder elektrisch bzw. elektronisch angesteuerten, eingestellten oder verstellten Mehrwegeventil oder Verteilerventil in der Dialysemaschine, kann der Fluss oder Strom von Dialysierflüssigkeit in der Schlauchleitung in einem vorbestimmten Bereich, beispielsweise zwischen 0 und 1000 ml/min, vorzugsweise zwischen 100 und 800 ml/min, jederzeit, beispielsweise auch im laufenden Betrieb oder Therapieprozess, verändert, variiert oder angepasst werden. Eine solche Veränderung, Variation oder Anpassung kann beispielsweise unter Rückkopplung von Temperaturen und/oder betriebsrelevanter Parameter, beispielsweise der Temperatur am Dialysatoreingang so erfolgen, dass die Temperatur der Dialysierflüssigkeit in einem gewünschten oder vorbestimmten Bereich bleibt oder innerhalb einer vorgegebenen Toleranz um einen Sollwert schwankt.

Vorteilhaft können weiter die zumindest zwei Lumen ebenfalls zumindest eine Wandung und/oder zumindest eine Isolationsstruktur wie vorstehend angegeben aufweisen.

Eine zusätzliche außenseitige Isolierung der Multi-Lumen-Schlauchleitung und/oder einzelner Lumen derselben kann die durch die mehreren Lumen bereits erzielten positiven Wirkungen auf den Dialysierflüssigkeitsfluss weiter verbessern. Da eine Blutreinigung einen therapeutisch und funktionell komplexen Gesamtvorgang involviert, ist es zur Erzielung einer Feinabstimmung denkbar, auch nur teilweise Abschnitte der Schlauchleitung, nur teilweise Abschnitte der Lumen in der Schlauchleitung, und/oder nur einzelne der vorhandenen Lumen zusätzlich zu isolieren. Denkbar ist prinzipiell jede mögliche, auch teilweise, Kombination der hierin beschriebenen Merkmale, Elemente und Funktionen, solange eine solche Kombination zu einer Verbesserung der Temperaturkonstanz am Dialysatoreingang beiträgt und im Übrigen den Dialyseprozess als solchen fördert oder günstig bzw. zumindest nicht unzumutbar nachteilig beeinflusst.

Bevorzugt ist bei der Dialysierflüssigkeits-Schlauchleitung zumindest dialysatorseitig eine Hansen-Kupplungsanordnung an einem Ende der Schlauchleitung vorgesehen.

Aufgrund der weiten Verbreitung von Hansen-Kupplungen im Bereich der Dialysebehandlung wird auf diese Weise gewährleistet, dass die Schlauchleitung mit üblicherweise verwendeten Dialysatoren kompatibel, verwendbar und unmittelbar einsatzbereit ist. Die Hansen-Kupplung erlaubt weiter auch den einfachen und kompatiblen Anschluss der Schlauchleitung an eine in der Dialysemaschine vorhandene Spülbrücke. Auch eine Nachrüstung bestehender Dialyseeinrichtungen ist somit leicht und effizient unter Aufrechterhaltung der bereits bestehenden Funktionalität möglich, und Schulungsaufwand kann entfallen.

### Kurzbeschreibung der Zeichnungen

Die Erfindung wird nachstehend anhand bevorzugter Ausführungsbeispiele unter Bezugnahme auf die beigefügte Zeichnung näher beschrieben. Es zeigen:
Fig. 1 in einer vereinfachten Darstellung eine Dialysemaschine, die durch eine Schlauchleitung für Dialysierflüssigkeit gemäß einem von sechs Ausführungsbeispielen mit einem Dialysator verbunden ist;
Fig. 2A bis 2C alternative Querschnitte der Schlauchleitung für Dialysierflüssigkeit gemäß einer Standard-Schlauchleitung (Fig. 2A), einem ersten (Fig. 2B) und einem zweiten (Fig. 2C) Ausführungsbeispiel der Schlauchleitung für Dialysierflüssigkeit; und
Fig. 3A bis 3D alternative Querschnitte der Schlauchleitung für Dialysierflüssigkeit gemäß einer Standard-Schlauchleitung (Fig. 3A), einem dritten (Fig. 3B), einem vierten (Fig. 3C) und einem fünften (Fig. 3D) Ausführungsbeispiel der Schlauchleitung für Dialysierflüssigkeit.

### Detaillierte Beschreibung bevorzugter Ausführungsbeispiele

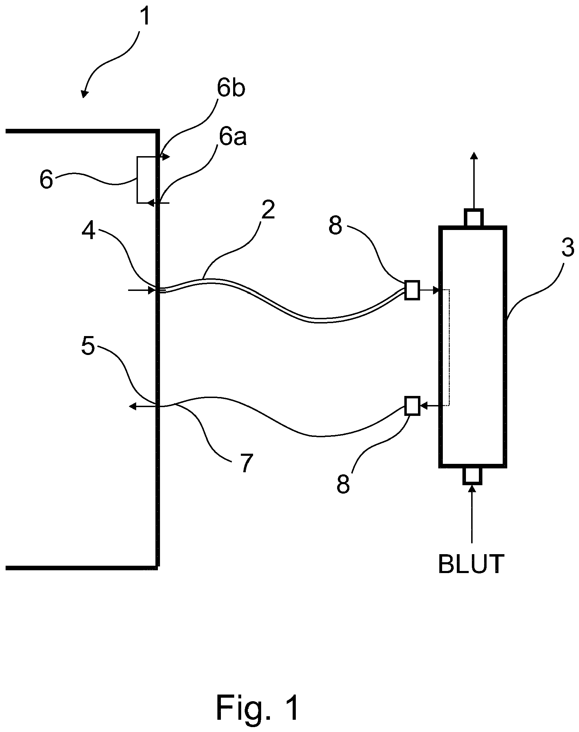

Fig. 1 zeigt in einer vereinfachten Darstellung eine Dialysemaschine 1, die durch eine erste Schlauchleitung (Schlauchzuführleitung) 2 für frische Dialysierflüssigkeit und eine zweite Schlauchleitung (Schlauchabführleitung) 7 für verbrauchte Dialysierflüssigkeit gemäß einem Ausführungsbeispiel mit einem Dialysator 3 verbunden ist.

Die Dialysemaschine 1 ist in Fig. 1 linksseitig angedeutet. Ein Dialysegerät bzw eine Dialysemaschine ist an sich bekannt, so dass Einzelheiten wie etwa interne Komponenten der Dialysemaschine 1, welche ohne direkten Bezug zu den Schlauchleitungen 2, 7 für Dialysierflüssigkeit sind, aus Gründen der Übersichtlichkeit nicht dargestellt sind.

In dem hier beschriebenen Ausführungsbeispiel haben, wie in Fig. 1 gezeigt, an der Dialysemaschine 1 insbesondere ein Dialysierflüssigkeits-Auslass bzw. -Ausgang 4, der einen Zufluss von frischer Dialysierflüssigkeit zu einem außerhalb der Dialysemaschine 1 angeordneten Dialysator 3 darstellt, ein Dialysierflüssigkeits-Einlass bzw. -Eingang 5, der einen Abfluss von verbrauchter Dialysierflüssigkeit aus dem Dialysator 3 bereitstellt, eine Spülbrücke 6 mit jeweils einem Einlass bzw. Eingang 6a und einem Auslass bzw. Ausgang 6b für Spülfluid, über welche die erste Schlauchleitung 2 für frische Dialysierflüssigkeit (Zufuhrleitung zum Dialysator 3) und die zweite Schlauchleitung 7 für verbrauchte Dialysierflüssigkeit (Abflussleitung vom Dialysator 3) zur Spülung und Desinfektion miteinander verbindbar (kurzschließbar) sind, näheren Bezug zu dem erfindungsgemäßen Schlauchquerschnitts-Konzept.

Hierbei wird unter frischer Dialysierflüssigkeit oder Dialysierlösung die frisch aufbereitete, noch unbelastete Dialysierflüssigkeit vor dem Dialysator 3 verstanden, und wird unter verbrauchter Dialysierflüssigkeit die mit harnpflichtigen Substanzen belastete Lösung, die den Dialysator 3 wieder verlässt und abfließt verstanden. Die hierin beschriebenen Schlauchquerschnitts-Konzepte sind grundsätzlich für beide Schlauchleitungen 2, 7 verwendbar, so dass auch dann, wenn eine Beschreibung nur im Hinblick auffrische Dialysierflüssigkeit erfolgt, in entsprechender Anordnung von Anschlüssen und gegebenenfalls Richtungsumkehr die Beschreibung auch grundlegend für verbrauchte Dialysierflüssigket Gültigkeit hat.

Der Anschluss der ersten Schlauchleitung 2 für frische Dialysierflüssigkeit an dem Dialysierflüssigkeits-Ausgang 4 der Dialysemaschine 1 und der Anschluss der Schlauchleitung 7 für verbrauchte Dialysierflüssigkeit an dem Dialysierflüssigkeits-Eingang 5 der Dialysemaschine 1 ist bevorzugt als fluiddichte Schlauchanbringung mit Austauschbarkeit von Schlauchverbindungen, beispielsweise für regelmäßige Wartung und/oder Erneuerung oder Nachrüstung, ausgebildet. Die Schlauchleitungen 2 und 7 weisen dialysatorseitig bevorzugt sogenannte Hansen-Kupplungen 8, eine an sich bekannte Vorrichtung zum schnellen Verbinden und Trennen von Fluidleitungen mit integrierten Absperrventilen, auf. Der Eingang 6a und der Ausgang 6b der Spülbrücke 6 können entsprechend zur Aufnahme der schlauchseitigen Hansen-Kupplungen vorbereitet sein.

Rechtsseitig in Fig. 1 ist der an sich ebenfalls bekannte Dialysator 3 angedeutet. Der Dialysator 3 ist die Schnittstelle zwischen einem extrakoporalen Blutkreislauf von einem Patienten und dem Dialysierflüssigkeits-Kreislauf der Dialysemaschine 1 und besteht beispielsweise aus einem Kunststoffgehäuse mit Hohlfaserbündeln oder Schichten von Membranen. In dem Dialysator 3 werden Dialysierflüssigkeit (in Fig. 1 an den Hansen-Kupplungen angedeutet) und Blut (in Fig. 1 unterseitig in den Dialysator 3 eintretend und oberseitig aus dem Dialysator 3 austretend angedeutet) gegenläufig geführt (Gegenstromprinzip), und erfolgt eine Filterung mit Stoffdurchtritt durch Diffusion an den semipermeablen Hohlfasern oder Membranen.

Nachstehend werden Ausführungsformen der ersten Schlauchleitung 2 und/oder der zweiten Schlauchleitung 7 mit mehreren Lumen bzw. Kanälen (Multi-Lumen-Schlauchleitung) näher beschrieben.

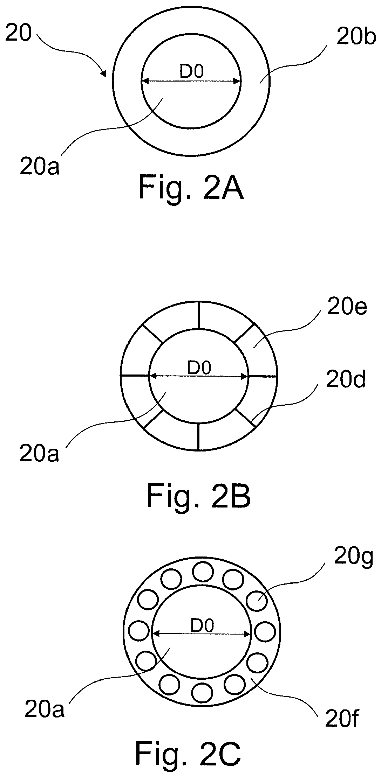

Fig. 2A bis 2C zeigen alternative Querschnitte der Schlauchleitung für Dialysierflüssigkeit gemäß einer Standard-Schlauchleitung (Fig. 2A), einem ersten (Fig. 2B) und einem zweiten (Fig. 2C) Ausführungsbeispiel der Schlauchleitung 2 für Dialysierflüssigkeit. Es ist möglich, die Schlauchleitung 2 entsprechend dem Ausführungsbeispiel nur für den Dialysierflüssigkeitszulauf von der Dialysemaschine 1 zu dem Dialysator 3 zu verwenden, oder optional ebenfalls die Schlauchleitung 7 dem Ausführungsbeispiel entsprechend auszuführen und für den Dialysierflüssigkeitsrücklauf von dem Dialysator 3 zu der Dialysemaschine 1 zu verwenden.

Die in Fig. 2A gezeigte Standard-Schlauchleitung für Dialysierflüssigkeit weist ein inneres Lumen bzw. einen inneren Fluidkanal 2a mit einem Durchmesser D0 und eine äußere Wandung oder Ummantelung 2b aus flexiblem, vorzugsweise transparentem oder opakem (milchig durchscheinendem) Material wie beispielsweise Silikon auf.

In einer ersten Modifikation der Schlauchleitung kann vorgesehen sein, den Durchmesser D0 gegenüber einem Standardwert von beispielsweise 5 mm auf 4 mm zu verringern. Dadurch wird die Flussgeschwindigkeit um mehr als 50% erhöht und gleichzeitig die Oberfläche um 20 % verringert, so dass sich rechnerisch entstehende Temperaturverluste nahezu halbieren. Da eine Querschnittsverringerung bei höheren Flussraten allerdings zu einem Druckanstieg von etwa 30 mBar bezogen auf 1 m Schlauchlänge und 800 ml/min führen kann, kann es im Hinblick auf geringstmöglichen Druckverlust und geringe Auswirkung auf Bilanzierungsergebnisse vorteilhaft sein, nur die zum Dialysator 3 hin führende Schlauchleitung 2 im Querschnitt zu verringern, um speziell dort die wichtige Temperaturkonstanz zu verbessern. Eine beispielhafte Modellrechnung erläutert dazu Größenordnungen und Verhältnisse:

| Innendurchmesser mm | Querschnitt mm² | Innenoberfläche mm² | Fluss ml/min | Geschwindigkeit m/min | Geschwindigkeit % | Fläche % | Verbesserung |
|---|---|---|---|---|---|---|---|
| 4 | 12,56 | 12,560 | 100,00 | 7,96 | 156 | 80 | 195 |
| 5 | 19,63 | 15,700 | 100.00 | 5,10 | 100 | 100 | |

In den in Fig. 2B und 2C gezeigten ersten und zweiten Ausführungsbeispielen beinhaltet die Schlauchleitung 20 für frische Dialysierflüssigkeit jeweils eine lufthaltige Isolierung gegen Wärmeübergang in die Umgebung, die außenseitig liegend ein von der Dialysierflüssigkeit durchströmtes inneres Lumen bzw. einen inneren (Fluid-)Kanal 20a der Schlauchleitung 2 umgibt. In anderen Worten fließt die Dialysierflüssigkeit in dem inneren, zentralen Lumen 20a durch die Schlauchleitung 20, und übt Luft in einer umliegenden Schicht oder Wandung bzw. einem umliegenden Schlauchabschnitt eine Isolationsfunktion aus.

Fig. 2B zeigt das erste Ausführungsbeispiel der Schlauchleitung 20 im Einzelnen. Die Isolation wird hier durch eine nicht voll ausgefüllte, kammerartige Außenstruktur der außenliegenden Wandung 20b bereitgestellt. Von der außenseitigen Oberfläche des inneren, zentralen Lumens (Hauptkanal) 20a mit dem Durchmesser D0 radial ausgehend und beispielsweise gleich beabstandet, aber nicht darauf beschränkt, sind stegartige Ausformungen (Schlauch-Längsleisten/Rippen) 20d bereitgestellt, die zu einer innenseitigen Oberfläche der äußeren Hülle der Schlauchleitung 20 führen. Die stegartigen Ausformungen bilden in der Außenstruktur der Schlauchleitung 20 luftgefüllte Kammern (Nebenkanäle) 20e aus. Die Isolationswirkung wird in diesem Fall durch die Luft in den Nebenkanälen 20e bereitgestellt, wobei die schmalen Stege als Kältebrücken einen nur geringen Wärmeübergang bewirken. Durch die nicht voll ausgefüllte Struktur bleibt vorteilhaft eine ausreichende Flexibilität der Schlauchleitung 20 erhalten, und ebenso vorteilhaft kann die Struktur aus bewährten Materialien, beispielsweise Silikon, extrudiert werden. Weiter vorteilhaft ist in diesem Fall die Schlauchleitung 20 transparent, so dass beispielsweise eine Rotfärbung durch Blutverlust und/oder Luftblasen in der Dialysierflüssigkeit leicht erkennbar sind.

Fig. 2C zeigt das zweite Ausführungsbeispiel der Schlauchleitung 20 im Einzelnen. In diesem Ausführungsbeispiel wird die kammerartige Außenstruktur durch in das Material 2f der Außenwandung der Schlauchleitung 20 eingebettete, beispielsweise kreisförmige und mit Luft gefüllte Nebenkanäle 20g gebildet. Bei transparentem Material 20f werden grundlegend dieselben Eigenschaften, Wirkungen und Vorteile wie bei dem in Fig. 2B gezeigten ersten Ausführungsbeispiel erzielt.

Bei allen in den Fig. 2A und 2C gezeigten Ausführungsformen der Schlauchleitung 20 ist es grundsätzlich möglich, zusätzlich eine isolierende Ummantelung vorzusehen. Des Weiteren ist es denkbar, die zunächst luftgefüllten Nebenkanäle als zuschaltbare Strömungskanäle vorzusehen. In anderen Worten ausgedrückt erfolgt die eigentliche Dialysierflüssigkeitsleitung durch den zentral angeordneten Hauptkanal. Reicht dessen Querschnitt jedoch nicht aus, um einen ausreichenden Fluidvolumenstrom mit akzeptabler Strömungsgeschwindigkeit zu fördern, können in ausgewählter Weise einzelne Nebenkanäle in die aktive Fluidleitung durch entsprechende Ventile am Auslass der Dialysemaschine oder am Einlass des Dialysators zugeschaltet werden, um den aktiven Strömungsquerschnitt zu vergößern.

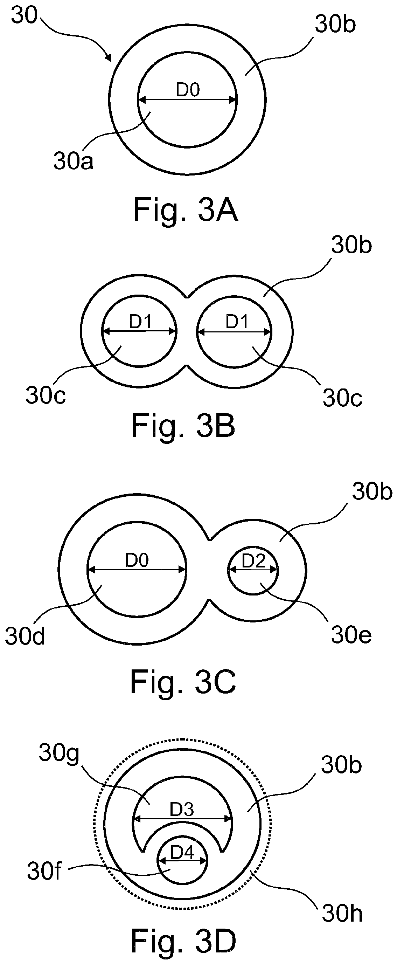

Fig. 3A bis 3D zeigen weiter alternative Querschnitte der Schlauchleitung 30 für Dialysierflüssigkeit gemäß der Standard-Schlauchleitung (Fig. 3A), einem dritten (Fig. 3B), einem vierten (Fig. 3C) und einem fünften (Fig. 3D) Ausführungsbeispiel der Schlauchleitung 30 für Dialysierflüssigkeit. Es ist auch bei diesen Ausführungsbeispielen möglich, die Schlauchleitung 2 entsprechend dem Ausführungsbeispiel nur für den Dialysierflüssigkeitstzulauf von der Dialysemaschine 1 zu dem Dialysator 3 zu verwenden, oder optional ebenfalls die Schlauchleitung 7 dem Ausführungsbeispiel entsprechend auszuführen und für den Dialysierflüssigkeitsrücklauf von dem Dialysator 3 zu der Dialysemaschine 1 zu verwenden.

Die in Fig. 3A gezeigte Standard-Schlauchleitung für Dialysierflüssigkeit weist ein inneres Lumen bzw. einen inneren Fluidkanal 30a mit einem Durchmesser D0 und eine äußere Wandung oder Ummantelung 30b aus flexiblem, vorzugsweise transparentem oder opakem (milchig durchscheinendem) Material wie beispielsweise Silikon auf.

Gemäß Fig. 3B ist in dem dritten Ausführungsbeispiel die Schlauchleitung 30 doppellumig mit jeweils gleichem Querschnitt bzw. Innendurchmesser D1 der beiden Einzellumen 30c ausgeformt. Der Innendurchmesser D1 jedes der Einzellumen 30c ist vorzugsweise kleiner als der Innendurchmesser D0 des Lumens der Standard-Schlauchleitung.

Gemäß Fig. 3C ist in dem vierten Ausführungsbeispiel die Schlauchleitung 30 doppellumig mit jeweils unterschiedlichen Querschnitten bzw. Innendurchmessern D0, D2 der beiden Einzellumen 30d, 30e ausgeformt. Der Innendurchmesser D2 des Einzellumens 30e ist vorzugsweise kleiner als der Innendurchmesser D0 des Lumens der Standard-Schlauchleitung, und weiter bevorzugt kleiner als der Innendurchmesser D1 des Einzellumens 30c der Schlauchleitung 30 gemäß dem dritten Ausführungsbeispiel.

Gemäß Fig. 3D ist in dem fünften Ausführungsbeispiel die Schlauchleitung 30 doppellumig so ineinander gearbeitet, dass ein kleineres Einzellumen 30f mit einem kleineren Innendurchmesser D4 durch eine innenliegende Wandung aus vorzugsweise demselben Material wie das der äußeren Wandung bzw. Ummantelung 30b getrennt in einem größeren Einzellumen 30g mit einem größeren Innendurchmesser bzw. Querschnitt D3 angeordnet ist. Der Innendurchmesser D4 des Einzellumens 30f ist vorzugsweise kleiner als der Innendurchmesser D0 des Lumens der Standard-Schlauchleitung, und weiter bevorzugt kleiner als der Innendurchmesser D1 des Einzellumens 30c der Schlauchleitung 30 gemäß dem dritten Ausführungsbeispiel. Im Hinblick auf beispielsweise Strömungs- und Druckverhältnisse kann der Innendurchmesser D4 gleich dem Innendurchmesser D2 des vierten Ausführungsbeispiels sein, und kann die Weite des Querschnitts bzw. der Innendurchmesser D3 näherungsweise gleich dem Innendurchmesser D0 der Standard-Schlauchleitung sein.

In den Fig. 3B bis 3D ist mithin eine Schlauchleitung 30 mit wenigstens zwei Lumen gezeigt, wobei grundsätzlich keine Beschränkung auf nur zwei Lumen besteht, und wobei die wenigstens zwei Lumen gleichen oder unterschiedlichen Durchmesser (Querschnittsfläche/Querschnittsform) aufweisen können. Für eine derartige Schlauchleitung 30 ist in der Dialysemaschine 1 vorzugsweise eine Mehrwege-Ventilvorrichtung, beispielsweise zwei 2/2-Wege-Ventile oder ein 3/2-Wege-Ventil angeordnet (nicht gezeigt), mittels der die Beaufschlagung der einzelnen Lumen mit Dialysierflüssigkeit wählbar ist. In anderen Worten kann mittels der Mehrwege-Ventilvorrichtung an der Dialysemaschine 1 gewählt werden, ob Dialysierflüssigkeit nur durch ein oder durch mehrere der vorhandenen Lumen fließt. An der Hansen-Kupplung 8 enden die wenigstens zwei Lumen und münden wieder in einen (Gesamt-) Dialyseflüssigkeitsstrom.

Eine Beschränkung auf eine bestimmte Lumenanzahl, beispielsweise zwei Lumen, oder bestimmte Beziehungen zwischen den einzelnen Lumenquerschnitten bzw. deren Innendurchmesser D0 bis D4 oder deren Maße besteht grundsätzlich nicht. Es ergeben sich jedoch bei Verwendung zur Durchleitung von Dialysierflüssigkeit im Rahmen eines Therapieprozesses zur Blutreinigung rechnerisch und praktisch vorteilhafte Kombinationen und/oder Größenverhältnisse.

So kann beispielsweise bei zwei Lumen gleichen Querschnitts bzw. Innendurchmessers D1 bei geringen Flussraten nur eines der beiden Lumen genutzt werden, und sind bei höheren Flussraten beide Lumen parallel schaltbar und nutzbar. Bei zwei Lumen unterschiedlichen Querschnitts bzw. Innendurchmessers D0, D2 kann beispielsweise bei geringen Flussraten, etwa in der Größenordnung von 100 ml/min bis etwa 300 ml/min, nur das Lumen mit dem kleineren Querschnitt bzw. Innendurchmesser D2, der dann beispielsweise 3 mm betragen kann, genutzt werden, während bei mittleren Flussraten, etwa in der Größenordnung von 300 ml/min bis etwa 500 ml/min, nur das Lumen mit dem größeren Querschnitt bzw. Innendurchmesser D0, der dann beispielsweise 5 mm betragen kann, genutzt wird, und bei höheren Flussraten, etwa in der Größenordnung von 500 ml/min bis etwa 800 ml/min, beide Lumen parallel geschaltet und gemeinsam zur Durchleitung von Dialysierflüssigkeit genutzt werden.

In einem Beispiel einer Schlauchleitung 2 mit zwei Lumen unterschiedlichen Querschnitts bzw. Innendurchmessers kann somit eine für die Durchleitung von Dialysierflüssigkeit zur Blutreinigung zweckmäßige und vorteilhafte Auslegung in einem Innendurchmesser von 5 mm für den größeren Querschnitt D0 und einem Innendurchmesser von 3 mm für den kleineren Querschnitt D2 bestehen. Diese Auslegung ist dahingehend vorteilhaft, dass bei höheren Flussraten gegenüber einer herkömmlichen Anordnung keine nachteiligen Effekte aufgrund von Druckverlusten auftreten, sondern sogar vorteilhaft der Druckverlust zu einem gewissen Grad verringert werden kann. Dennoch kann bei geringen Flussraten der Temperaturverlust in die Umgebung klein gehalten werden.

Eine beispielhafte Modellrechnung erläutert dazu Größenordnungen und Verhältnisse:

| Innendurchmesser mm | Querschnitt mm² | Geschwindigkeit bei 100 ml/min in m/min | Zeit für 1 m in s | Druckverlust in mBar bei 1 m Länge und 100 ml/min | Druckverlust in mBar bei 1 m Länge und 800 ml/min |
|---|---|---|---|---|---|
| 3 | 7,1 | 14,15 | 4,24 | 8,4 | 216,17 |
| 5 | 19,6 | 5,09 | 11,78 | 1,09 | 19,4 |

Während eines Desinfektionsprozesses kann vorteilhaft ein Fluss von Dialysierflüssigkeit mehrmals abwechselnd durch die einzelnen Lumen kleineren und größeren Durchmessers D0 und D2, oder alternativ durch beide dieser Lumen gleichzeitig erzeugt werden.

Es wird angemerkt, dass wie in Fig. 3D mittels durchbrochener Linie angedeutet, jedes der zweiten bis fünften Ausführungsbeispiele zusätzlich mit einer isolierenden Ummantelung (entsprechend der Ummantelung 30c des ersten Ausführungsbeispiels) versehen sein kann, und sich damit durch Kombination eine isolierte Multi-Lumen-Schlauchleitung 30 für Dialysierflüssigkeit und/oder Dialysat zur Verwendung im Hinlauf zum Dialysator 3 und/oder im Rücklauf zu der Dialysemaschine 1 als optimale Gesamtlösung darstellen lässt.

### Bezugszeichenliste

- 1: Dialysemaschine
- 2: Schlauchleitung Dialysierflüssigkeit
- 3: Dialysator
- 4: Dialysierflüssigkeits-Ausgang
- 5: Eingang verbrauchte Dialysierflüssigkeit
- 6: Spülbrücke
- 6a: Eingang Spülbrücke
- 6b: Ausgang Spülbrücke
- 7: Schlauchleitung verbrauchte Dialysierflüssigkeit
- 8: Hansen-Kupplung

- 20: Schlauchleitung
- 20a: Lumen, Fluidkanal
- 20b: Wandung
- 20d: stegartige Ausformung
- 20e: kammerförmige luftgefüllte Kanalstruktur
- 20f: Material Außenwandung
- 20g: kreisförmige luftgefüllte Kanalstruktur

- 30: Schlauchleitung
- 30a: Lumen, Fluidkanal
- 30b: Wandung
- 30c, 30d, 30e, 30f, 30g: Einzellumen bei Multi-Lumen-Schlauchleitung
- 30h: Isolationsstruktur

- D0, D1, D2, D3, D4: Durchmesser Lumen

## Patentansprüche

1. Dialysierflüssigkeits-Schlauchleitung (2; 20; 30) zur Herstellung einer Fluidverbindung zwischen einem Dialysierflüssigkeits-Ausgang (4) an einer Dialysemaschine (1) und einem Dialysierflüssigkeits-Eingang an einem Dialysator (3) und/oder zur Herstellung einer Fluidverbindung zwischen einem Dialysierflüssigkeits-Ausgang an dem Dialysator (3) und einem Dialysierflüssigkeits-Eingang (5) an der Dialysemaschine (1), **gekennzeichnet durch**
eine mehrlumige Schlauchquerschnittsgeometrie, die zusätzlich zu einem Hauptlumen (20a, 30a, 30c, 30d, 30g) in der Funktion als Hauptströmungskanal wenigstens ein weiteres Nebenlumen (20e, 20g, 30c, 30e, 30f) in der Funktion als Isolationskammer zur umfangsseitigen Wärmeisolation des Hauptlumens und in der Funktion als Nebenströmungskanal für die wahlweise alternative oder zusätzliche Dialysierflüssigkeitsförderung in die gleiche Richtung wie das Hauptlumen vorsieht, wobei an einem Ende der Schlauchleitung eine Vorrichtung angeordnet ist, mit der ein Dialysierflüssigkeitsfluss auf nur ein Lumen oder auf parallel mehrere Lumen bis hin zu allen Lumen der Schlauchleitung schaltbar ist, wodurch der Dialysierflüssigkeitsfluss in der Schlauchleitung in einem vorbestimmten Bereich jederzeit veränderbar, variierbar oder anpassbar ist.

2. Dialysierflüssigkeits-Schlauchleitung nach Anspruch 1, **dadurch gekennzeichnet, dass** wenigstens das Hauptlumen (20a; 30a, 30c bis 30g) eine Wandung (20b, 30b) aus einem flexiblen und/oder transparentem oder opaken Material aufweist, und die Wandung (20b; 30b) außenseitig von einer Isolationsstruktur (30h) umgeben ist, in welcher Luft einen Wärmeübergang aus der Dialysierflüssigkeit in die Umgebung der Schlauchleitung (2; 20; 30) verringert.

3. Dialysierflüssigkeits-Schlauchleitung nach Anspruch 2, **dadurch gekennzeichnet, dass** die Isolationsstruktur (30h) aus einem extrudierten und/oder geschäumten, flexiblen Material oder aus demselben Material wie die Wandung (20b; 30b) des wenigstens einen Lumens (20a; 30a, 30c bis 30g) besteht.

4. Dialysierflüssigkeits-Schlauchleitung nach Anspruch 3, **dadurch gekennzeichnet, dass** die Isolationsstruktur (30h) kammerförmige (20e) oder kreisförmige (20g), zumindest temporär luftgefüllte Kanalstrukturen (20e, 20g) als die Nebenlumen aufweist, die durch stegartige, radial nach außen verlaufende, sowie in Umfangsrichtung beabstandete Längsrippen (20f) der Wandung (20b, 30b) und/oder der Isolationsstruktur (30h) voneinander getrennt und äquidistant in der Isolationsstruktur (30h) verteilt sind.

5. Dialysierflüssigkeits-Schlauchleitung nach einem der vorstehenden Ansprüche, **dadurch gekennzeichnet, dass**
- das Hauptlumen (30c, 30d, 30g) und das wenigstens eine weitere Nebenlumen (30c, 30e, 30f) gleiche oder unterschiedliche Querschnitte und/oder gleiche oder unterschiedliche Querschnittsflächenmaße aufweisen.

6. Dialysierflüssigkeits-Schlauchleitung nach einem der vorstehenden Ansprüche, **dadurch gekennzeichnet, dass** wenigstens eines der Lumen, vorzugsweise das Nebenlumen ein anderes Lumen, vorzugsweise das Hauptlumen zumindest über einen Teilkreisabschnitt hinweg umgibt, oder umgekehrt.

7. Dialysierflüssigkeits-Schlauchleitung nach einem der vorstehenden Ansprüche, **dadurch gekennzeichnet, dass** die wenigstens zwei Lumen (30g, 30f) jeweils unterschiedlichen Querschnitt und/oder Durchmesser (D3, D4) aufweisen und dafür vorgesehen sind, einzeln für einen Dialysierflüssigkeitsfluss einer dem jeweiligen Einzellumen entsprechenden Rate oder parallel gleichzeitig für einen Dialysierflüssigkeitsfluss einer dem Gesamtvolumen entsprechenden Rate schaltbar zu sein.

8. Dialysemaschine (1) mit einem außerhalb der Maschine angeordneten Dialysator (3) sowie zwei Dialysierflüssigkeitsleitungen (2, 7), die an einen Einlass und einen Auslass der Dialysators (3) angeschlossen oder anschließbar sind, um den Dialysator (3) mit der Dialysiermaschine (1) fluidzuverbinden, **dadurch gekennzeichnet, dass** zumindest der an den Dialysatoreinlass angeschlossene oder anschließbare Dialysierflüssigkeitsschlauch eine Dialysierflüssigkeits-Schlauchleitung gemäß einem der Ansprüche 1 bis 7 aufweist.

9. Dialysemaschine (1) nach Anspruch 8, **dadurch gekennzeichnet, dass** der an den Dialysatoreinlass angeschlossene oder anschließbare Dialysierflüssigkeitsschlauch (2) einen Strömungsquerschnitt hat, der größer ist als der Strömungsquerschnitt des an den Dialysatorauslass angeschlossenen oder anschließbaren Dialysierflüssigkeitsschlauchs (7).

10. Dialysemaschine (1) gemäß Anspruch 9, **dadurch gekennzeichnet, dass** die wenigstens zwei Lumen (30c bis 30g) bei Beaufschlagung mit Dialysierflüssigkeit von einer Schlauch-eingangsseitig angeordneten Schaltvorrichtung einzeln oder in paralleler Weise für die Beaufschlagung mit einem Dialysierflüssigkeitsfluss freischaltbar sind, und vorzugsweise Schlauch-ausgangsseitig in ein Einzellumen münden.

11. Dialysemaschine (1) nach einem der Ansprüche 8 bis 10, wobei die Vorrichtung ein manuell oder elektrisch bzw. elektronisch angesteuertes, eingestelltes oder verstelltes Mehrwegeventil oder Verteilerventil in der Dialysemaschine (1) ist.

## Claims

1. Dialysis fluid hose line (2; 20; 30) for establishing a fluid connection between a dialysis fluid outlet (4) on a dialysis machine (1) and a dialysis fluid inlet on a dialyzer (3) and/or for establishing a fluid connection between a dialysis fluid outlet on the dialyzer (3) and a dialysis fluid inlet (5) on the dialysis machine (1), **characterised by**
a multi-lumen hose cross-sectional geometry providing at least one additional side lumen (20e, 20g, 30c, 30e, 30f) functioning as isolation chamber in addition to a main lumen (20a, 30a, 30c, 30d, 30g) functioning as main flow channel for the circumferential heat insulation of the main lumen and functioning as side flow channel for the optionally alternative or additional dialysis fluid delivery in the same direction as the main lumen, wherein at one end of the hose line a device is arranged for switching a dialysis fluid flow to just one lumen or in parallel to several lumens up to all lumens of the hose line, whereby the dialysis fluid flow in the hose line can be altered, varied or adjusted in a predetermined area at anytime.

2. Dialysis fluid hose line according to claim 1, **characterised in that** at least the main lumen (20a; 30a, 30c to 30g) comprises a wall (20b, 30b) of a flexible and/or transparent or opaque material, and the wall (20b; 30b) is surrounded on the outside by an insulating structure (30h) in which air reduces a heat transfer from the dialysis fluid into the surrounding of the hose line (2; 20; 30).

3. Dialysis fluid hose line according to claim 2, **characterised in that** the insulating structure (30h) is made of an extruded and/or foamed flexible material or of the same material as the wall (20b; 30b) of the at least one lumen (20a; 30a, 30c to 30g).

4. Dialysis fluid hose line according to claim 3, **characterised in that** the insulating structure (30h) comprises as the side lumens chamber-form (20e) or circular (20g) channel structures (20e, 20g) which are at least temporarily filled with air, and which are separated from one another by bar-shaped longitudinal ribs (20f) of the wall (20b, 30b) and/or the insulating structure (30h), the ribs extending radially outwards and being spaced apart from each other in the circumferential direction and being equidistantly distributed in the insulating structure (30h).

5. Dialysis fluid hose line according to any of the preceding claims, **characterised in that**
- the main lumen (30c, 30d, 30g) and the at least one additional side lumen (30c, 30e, 30f) comprise the same or different cross-sections and/or comprise the same or different cross-sectional diameters.

6. Dialysis fluid hose line according to any of the preceding claims, **characterised in that** at least one of the lumens, preferably the side lumen, surrounds another lumen, preferably the main lumen, over at least a pitch circle section, or vice versa.

7. Dialysis fluid hose line according to any of the preceding claims, **characterised in that** the at least two lumens (30g, 30f) each have a different cross-section and/or diameter (D3, D4) and are configured to be switched separately for a dialysis fluid flow at a rate corresponding to the respective single lumen or to be switched at the same time in parallel for a dialysis fluid flow a rate corresponding to the total volume.

8. Dialysis machine (1) with a dialyzer (3) arranged outside the machine and two dialysis fluid lines (2, 7) connected or connectable to an inlet and an outlet of the dialyzer (3) to fluidly connect the dialyzer (3) to the dialysis machine (1), **characterised in that** at least the dialysis fluid hose connected or connectable to the dialyzer inlet comprises a dialysis fluid hose line according to any of claims 1 to 7.

9. Dialysis machine (1) according to claim 8, **characterised in that** the dialysis fluid hose (2) connected or connectable to the dialyzer inlet has a flow cross-section that is larger than the flow cross-section of the dialysis fluid hose (7) connected or connectable to the dialyzer outlet.

10. Dialysis machine (1) according to claim 9, **characterised in that** the at least two lumens (30c to 30g), when charged with dialysis fluid, can separately or in parallel be activated by a switching device arranged at the inlet side of the hose for charging with a dialysis fluid flow, and preferably open into a single lumen at the outlet side of the hose.

11. Dialysis machine (1) according to any of claims 8 to 10, wherein the device is a multi-port valve or distribution valve in the dialysis machine (1) which is manually or electrically or electronically controlled, set or adjusted.

## Revendications

1. Tuyau souple de liquide de dialyse (2 ; 20 ; 30) pour l'établissement d'une liaison de fluide entre une sortie de liquide de dialyse (4) sur une machine de dialyse (1) et une entrée de liquide de dialyse sur un dialyseur (3) et/ou pour l'établissement d'une liaison de fluide entre une sortie de liquide de dialyse sur le dialyseur (3) et une entrée de liquide de dialyse (5) sur la machine de dialyse (1), **caractérisé par**
une géométrie de section transversale de tuyau à plusieurs lumens qui prévoit outre un lumen principal (20a, 30a, 30c, 30d, 30g) fonctionnant comme canal d'écoulement principal au moins un autre lumen accessoire (20e, 20g, 30c, 30e, 30f) fonctionnant comme chambre d'isolation pour l'isolation thermique côté périphérique du lumen principal et fonctionnant comme canal d'écoulement accessoire pour le transport de liquide de dialyse alternatif ou supplémentaire au choix dans la même direction que le lumen principal, dans lequel un dispositif est agencé sur une extrémité du tuyau souple, avec lequel un flux de liquide de dialyse peut être commuté sur un seul lumen ou sur plusieurs lumens parallèles jusqu'à tous les lumens du tuyau souple, par quoi le flux de liquide de dialyse peut être modifié, varié ou adapté à tout moment dans une zone prédéterminée.

2. Tuyau souple de liquide de dialyse selon la revendication 1, **caractérisé en ce qu'au** moins le lumen principal (20a ; 30a, 30c à 30g) présente une paroi (20b, 30b) en un matériau souple et/ou transparent ou opaque, et la paroi (20b ; 30b) est entourée côté extérieur par une structure d'isolation (30h), dans laquelle l'air réduit un transfert de chaleur du liquide de dialyse dans l'environnement du tuyau souple (2 ; 20 ; 30).

3. Tuyau souple de liquide de dialyse selon la revendication 2, **caractérisé en ce que** la structure d'isolation (30h) se compose d'un matériau souple extrudé et/ou expansé, ou du même matériau que la paroi (20b ; 30b) d'au moins un lumen (20a ; 30a, 30c à 30g).

4. Tuyau souple de liquide de dialyse selon la revendication 3, **caractérisé en ce que** la structure d'isolation (30h) présente des structures de canal (20e, 20g) en forme de chambre (20e) ou circulaires (20g), remplies d'air au moins temporairement comme les lumens accessoires qui sont séparés les uns des autres par des nervures longitudinales (20f) de type barrette, s'étendant radialement vers l'extérieur, ainsi qu'espacées dans le sens périphérique de la paroi (20b, 30b) et/ou la structure d'isolation (30h) et sont répartis à équidistance dans la structure d'isolation (30h).

5. Tuyau souple de liquide de dialyse selon l'une quelconque des revendications précédentes, **caractérisé en ce que**
- le lumen principal (30c, 30d, 30g) et l'au moins un autre lumen accessoire (30c, 30e, 30f) présentent des sections transversales identiques ou différentes et/ou des mesures de superficie de section identiques ou différentes.

6. Tuyau souple de liquide de dialyse selon l'une quelconque des revendications précédentes, **caractérisé en ce qu'**au moins un des lumens, de préférence le lumen accessoire entoure un autre lumen, de préférence le lumen principal au moins au-delà d'une section circulaire partielle, ou inversement.

7. Tuyau souple de liquide de dialyse selon l'une quelconque des revendications précédentes, **caractérisé en ce que** les au moins deux lumens (30g, 30f) présentent respectivement une différente section transversale et/ou un différent diamètre (D3, D4) et sont prévus afin d'être commutables individuellement pour un flux de liquide de dialyse d'un taux correspondant au lumen individuel respectif ou parallèlement simultanément pour un flux de liquide de dialyse d'un taux correspondant au volume global.

8. Machine de dialyse (1) avec un dialyseur (3) agencé en dehors de la machine ainsi que deux tuyaux de liquide de dialyse (2, 7) qui sont raccordés ou peuvent être raccordés à une entrée et une sortie du dialyseur (3) afin de relier par voie fluide le dialyseur (3) à la machine de dialyse (1), **caractérisée en ce qu'**au moins le tuyau de liquide de dialyse raccordé ou pouvant être raccordé à l'entrée de dialyse présente un tuyau souple de liquide de dialyse selon l'une des revendications 1 à 7.

9. Machine de dialyse (1) selon la revendication 8, **caractérisée en ce que** le tuyau de liquide de dialyse (2) raccordé ou pouvant être raccordé à l'entrée de dialyseur présente une section transversale d'écoulement qui est supérieure à la section transversale d'écoulement du tuyau de liquide de dialyse (7) raccordé ou pouvant être raccordé à la sortie de dialyseur.

10. Machine de dialyse (1) selon la revendication 9, **caractérisée en ce que** les au moins deux lumens (30c à 30g) peuvent être déverrouillés en cas d'alimentation en liquide de dialyse par un dispositif de commutation agencé côté entrée de tuyau individuellement ou parallèlement pour l'alimentation en un flux de liquide de dialyse, et débouchent de préférence coté sortie de tuyau dans un lumen individuel.

11. Machine de dialyse (1) selon l'une des revendications 8 à 10, dans laquelle le dispositif est une soupape à plusieurs voies ou soupape de distribution commandée, réglée ou déplacée manuellement ou électriquement ou électroniquement dans la machine de dialyse (1).
